# EUROPEAN PATENT SPECIFICATION

(11) **EP 2 188 286 B1**
(45) Date of publication and mention of the grant of the patent: **18.04.2012**
(21) Application number: 08787335.2
(22) Date of filing: 20.08.2008
(51) Int. Cl.: C07D 471/04, A61K 31/4375, A61P 3/00

(54) **PROCESS FOR THE PREPARATION OF PYRIDO [2, 1-A]ISOQUINOLINE DERIVATIVES**
VERFAHREN ZUR HERSTELLUNG VON PYRIDO[2,1-A]ISOCHINOLINDERIVATEN
PROCÉDÉ DE PRÉPARATION DE DÉRIVÉS DE PYRIDO[2,1-A]ISOQUINOLÉINE

(30) Priority: 30.08.2007 EP 07115302
(43) Date of publication of application: 26.05.2010
(73) Proprietor: F. Hoffmann-La Roche AG, 4070 Basel (CH)
(72) Inventor: BROMBERGER, Ulrike, CH-4123 Allschwil (CH); DIODONE, Ralph, 79206 Breisach (DE); HILDBRAND, Stefan, 4460 Gelterkinden (CH); MEIER, Roland, CH-5028 Ueken (CH)
(74) Representative: Waschbüsch, Klaus
(86) International application number: PCT/EP2008/060865
(87) International publication number: WO 2009/027276

(56) References cited:
- WO-A-2005/000848
- WO-A-2006/125728
- COFFEY, D.S. ET AL.: ORGANIC PROCESS AND DEVELOPMENT, vol. 8, 2004, pages 945-947, XP002502673
- ATTANASI, O.A. ET AL.;: J. CHEM. SOC. PERKIN TRANS. 1, 1997, pages 1829-1835, XP002502674

## Description

The present invention is concerned with a novel process for the preparation of pyrido[2,1-a]isoquinoline derivatives. The pyrido[2,1-a]isoquinoline derivatives of formula I 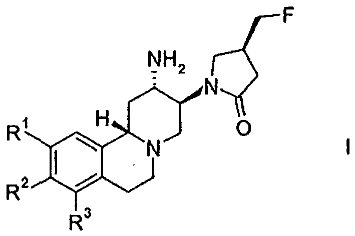 wherein R¹, R² and R³ are each independently hydrogen, halogen, hydroxy, lower alkyl, lower alkoxy or lower alkenyl, wherein lower alkyl, lower alkoxy and lower alkenyl may optionally be substituted by lower alkoxycarbonyl, aryl or heterocyclyl and the pharmaceutically acceptable salts thereof are useful for the treatment and/or prophylaxis of diabetes, particularly non-insulin dependent diabetes mellitus, and/or impaired glucose tolerance, as well as other conditions wherein the amplification of action of a peptide normally inactivated by DPP-IV gives a therapeutic benefit. The compounds can also be used in the treatment and/or prophylaxis of obesity, inflammatory bowel disease, Colitis Ulcerosa, Morbus Crohn, and/or metabolic syndrome or β-cell protection. Furthermore, the compounds can be used as diuretic agents and for the treatment and/or prophylaxis of hypertension (PCT Publication No. WO 2005/000848).

The process for preparation as described in the PCT Publication No. WO 2005/000848 comprises a reaction sequence which suffers from a number of disadvantages which prevents this process from being useful on technical scale. Particularly it was found that the coupling reaction in the presence of 2-hydroxypyridine as catalyst under the conditions outlined in WO 2005/000848 led to a comparable lower conversion which necessitates an intermediate isolation step and that the deprotection of the N-protected pyrido[2,1-a]isoquinoline derivative of formula II with acetyl chloride or hydrogen chloride in aliphatic alcohols led to toxic alkylchloride by-products.

WO 2006/125728 describes a two step process for the preparation of compound of formula II from compound of formula III with the isolation of compound of formula V.

The object of the present invention therefore was to develop a process which avoids the disadvantages found in the prior art process and which is applicable on technical scale.

The object has been achieved with the process as outlined below.

The process of the present invention comprises the preparation of a pyrido[2,1-a]isoquinoline derivative of formula I 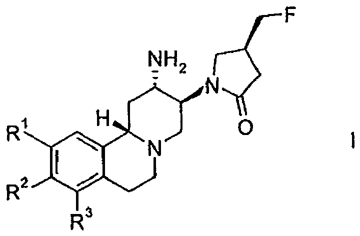 wherein R¹, R² and R³ are each independently hydrogen, halogen, hydroxy, lower alkyl, lower alkoxy or lower alkenyl, wherein lower alkyl, lower alkoxy and lower alkenyl may optionally be substituted by lower alkoxycarbonyl, aryl or heterocyclyl, and pharmaceutically acceptable salts thereof,
comprising
a) coupling an amine of formula III 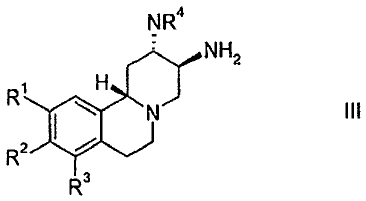 wherein R¹, R² and R³ are each independently hydrogen, halogen, hydroxy, lower alkyl, lower alkoxy or lower alkenyl, wherein lower alkyl, lower alkoxy and lower alkenyl may optionally be substituted by lower alkoxycarbonyl, aryl or heterocyclyl and R⁴ is an amino protecting group, with the fluorolactone of the formula IV 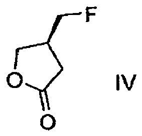 in the presence of 6-chloropyridine-2-ol as catalyst to form the butyramide of the formula V 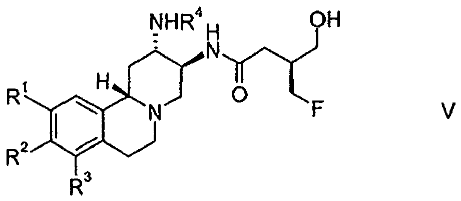 wherein R¹, R², R³ and R⁴ are as defined above;
b) forming the mesylate of formula VI 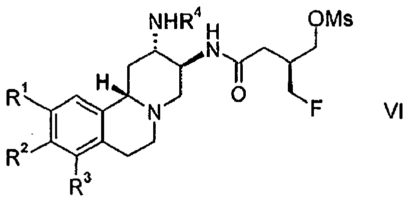 wherein R¹, R², R³ and R⁴ are as defined above and Ms stands for methanesulfonyl, by reacting the butyramide of formula V with a methanesulfonyl chloride or methanesulfonyl anhydride;
c) ring closing of the mesylate of formula VI in the presence of an organic base to form the N-protected pyrido[2,1-a]isoquinoline derivative of formula II 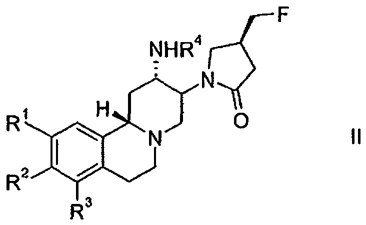 wherein R¹, R² and R³ are as defined above and R⁴ is an acid-labile amino protecting group and
d) deprotecting the N-protected pyrido[2,1-a]isoquinoline derivative of formula II with hydrochloric acid in a solvent selected from an aliphatic ketone, an aliphatic nitrile or a cyclic ether or mixtures thereof or in water in admixture with an aliphatic ketone, an aliphatic nitrile or a cyclic ether or mixtures thereof;
   wherein at the end of step a), the amount of solvent must be reduced by distillation in order to achieve full conversion of compound of amine of formula III to butyramide of formula V,
   wherein the butyramide of formula V obtained from step a) is used for the mesylation step b) without isolation from the reaction mixture.

Unless otherwise indicated, the following definitions are set forth to illustrate and define the meaning and scope of the various terms used to describe the invention herein.

In this specification the term "lower" is used to mean a group consisting of one to six, preferably of one to four carbon atom(s).

The term "halogen" refers to fluorine, chlorine, bromine and iodine, with fluorine, bromine and chlorine being preferred.

The term "lower alkyl", alone or in combination with other groups, refers to a branched or straight-chain monovalent alkyl radical of one to six carbon atoms, preferably one to four carbon atoms. This term is further exemplified by radicals such as methyl, ethyl, n-propyl, isopropyl, n-butyl, s-butyl, isobutyl, t-butyl, n-pentyl, 3-methylbutyl, n-hexyl, 2-ethylbutyl and the like. Preferable lower alkyl residues are methyl and ethyl, with methyl being especially preferred.

The term "lower alkoxy" refers to the group R'-O-, wherein R' is lower alkyl as defined herein before. Examples of lower alkoxy groups are e.g. methoxy, ethoxy, propoxy, isopropoxy, butoxy, isobutoxy and hexyloxy, with methoxy being especially preferred.

The term "lower alkoxycarbonyl" refers to the group R'-O-C(O)-, wherein R' is lower alkyl.

The term "aryl" refers to an aromatic monovalent mono- or polycarbocyclic radical, such as phenyl or naphthyl, preferably phenyl, which may optionally be mono-, di- or tri-substituted, independently, by lower alkyl, lower alkoxy, halo, cyano, azido, amino, di-lower alkyl amino or hydroxy.

The term "heterocyclyl" refers to a 5- or 6-membered aromatic or saturated N-heterocyclic residue, which may optionally contain a further nitrogen or oxygen atom, such as imidazolyl, pyrazolyl, thiazolyl, pyridyl, pyrimidyl, morpholino, piperazino, piperidino or pyrrolidino, preferably pyridyl, thiazolyl or morpholino. Such heterocyclic rings may optionally be mono-, di- or tri-substituted, independently, by lower alkyl, lower alkoxy, halo, cyano, azido, amino, di-lower alkyl amino or hydroxy. Preferable substituent is lower alkyl, with methyl being preferred.

The term "acid-labile amino protecting group" refers to any substituents conventionally used to hinder the reactivity of the amino group that can be cleaved with an acid such as hydrochloric acid. Suitable amino protecting groups are selected from the group consisting of the formyl group, amide groups, and carbamate groups such as 4-methoxybenzyloxycarbonyl ("Moz") and tert-butoxycarbonyl ("Boc"). The selection and use (addition and subsequent removal) of amino protecting groups is well known to the skilled in the art. Further examples of groups referred to by the above terms are described by T. W. Greene and P. G. M. Wuts, Protective Groups in Organic Synthesis, 3rd edition, John Wiley and Sons, New York, NY, 1999. Preferred amino protecting group for the process of the present invention is Boc.

The term "pharmaceutically acceptable salts" embraces salts of the compounds of formula (I) with inorganic or organic acids such as hydrochloric acid, hydrobromic acid, nitric acid, sulphuric acid, phosphoric acid, citric acid, formic acid, maleic acid, acetic acid, fumaric acid, succinic acid, tartaric acid, methanesulphonic acid, salicylic acid, p-toluenesulphonic acid and the like, which are non toxic to living organisms. Preferred salts with acids are formates, maleates, citrates, hydrochlorides, hydrobromides and methanesulfonic acid salts, with hydrochlorides being especially preferred.

The processes of the present invention are described in more detail below.

### Step a)

Step a) comprises the coupling an amine of formula III 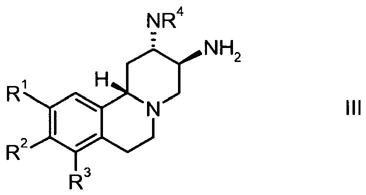 wherein R¹, R² and R³ are each independently hydrogen, halogen, hydroxy, lower alkyl, lower alkoxy, or lower alkenyl, wherein lower alkyl, lower alkoxy and lower alkenyl may optionally be substituted by lower alkoxycarbonyl, aryl or heterocyclyl and R⁴ is an acid-labile amino protecting group, with the fluorolactone of the formula IV 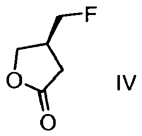 in the presence of 6-chloropyridine-2-ol as catalyst to form the butyramide of the formula V 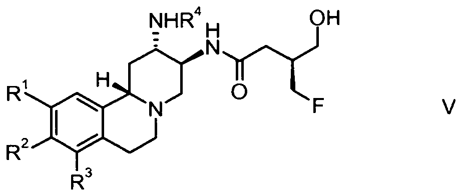 wherein R¹, R², R³ and R⁴ are as defined above.

The amine of formula III can be prepared following the methods described in the PCT Publication No. WO 2005/000848, particularly as described in scheme 3.

In a preferred embodiment of the present invention, R¹ and R² have the meaning of a methoxy group, R³ is hydrogen and R⁴ signifies a Boc group.

The 6-chloropyridin-2-ol catalyst is usually applied in an amount of 0.05 mol equivalents to 0.20 mol equivalents relating to one mol equivalent of the amine of formula III.

The coupling reaction is as a rule performed in a suitable organic solvent such as in toluene or mixtures thereof, preferably in toluene at a temperature of 80 °C to 111 °C.

At the end of the reaction, the amount of solvent must be reduced by distillation in order to achieve a full conversion. Upon completion of the conversion the butyramide of formula IV can be used for the mesylation step b) without its isolation from the reaction mixture.

### Step b)

Step b) requires forming of the mesylate of formula VI 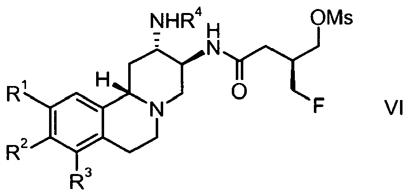 wherein R¹, R², R³ and R⁴ are as defined above and Ms stands for methanesulfonyl, by reacting the butyramide of formula V with a methanesulfonyl chloride or methanesulfonyl anhydride.

In a preferred embodiment of the present invention, R¹ and R² have the meaning of a methoxy group, R³ is hydrogen and R⁴ signifies a Boc group.

As outlined above the reaction mixture resulting in step a), is diluted with THF or dioxane and then directly used for the mesylation in step b).

Preferably, the reaction is carried out with methanesulfonyl chloride.

The reaction is expediently performed in an organic solvent, such as in tetrahydrofuran (THF), dioxane or mixtures of THF or dioxane with toluene, preferably in a mixture of THF and toluene, at a temperature of 10 °C to 35 °C.

An amine, such as N-methylmorpholine or a tertiary aliphatic amine, preferably triethylamine or tributylamine, should be present as well to absorb the HCl generated. Most preferably, triethylamine is used.

Upon completion of the reaction the mesylate of formula VI can be used for the ring closing step c) without its isolation from the reaction mixture.

### Step c)

In step c) the ring closing of the mesylate of formula VI in the presence of an organic base takes place to form the N-protected pyrido[2,1-a]isoquinoline derivative of formula II 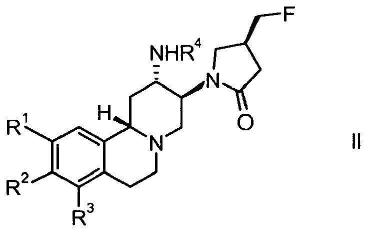 wherein R¹, R² and R³ are as defined above and R⁴ is an acid-labile amino protecting group.

In a preferred embodiment of the present invention R¹ and R² have the meaning of a methoxy group, R³ is hydrogen and R⁴ signifies a Boc group.

As outlined above the reaction mixture resulting in step b) can directly be used for the ring closing reaction in step c).

The organic base used for the ring closing reaction can be selected from lithium tert.-butoxide, sodium tert.-butoxide or potassium tert.-butoxide, lithium-bis (trimethylsilyl) amide (LHMDS, lithium hexamethyldisilazane), n-butyl-lithium (n-BuLi) or lithium diisopropylamide (LDA). Preferred organic bases are lithium-bis(trimethylsilyl)amide and lithium tert.-butoxide which are as a rule applied in an amount of 2.5 mol equivalents to 3.5 mol equivalents relating to one mol equivalent of the amine of formula III.

The reaction is customarily performed in an organic solvent such as in tetrahydrofuran (THF) or dioxane or their mixtures with toluene, preferably in a mixture of THF and toluene or a mixture of dioxane and toluene at a temperature of -20 °C to 10 °C.

The isolation of the N-protected pyrido[2,1-a]isoquinoline derivative of formula II can happen by applying techniques well known to the skilled in the art e.g. by quenching of the reaction mixtures with water, separation of the organic phase and subsequent crystallization through solvent change to an aliphatic alcohol e.g. to methanol.

### Step d)

Step d comprises deprotecting of the N-protected pyrido[2,1-a]isoquinoline derivative of formula II with hydrochloric acid in a solvent selected from an aliphatic ketone, an aliphatic nitrile or a cyclic ether or mixtures thereof or water in admixture with an aliphatic ketone, an aliphatic nitrile or a cyclic ether or mixtures thereof.

Preferably the solvent is selected from acetone, methyl ethyl ketone, acetonitrile or tetrahydrofuran or mixtures thereof or from water in admixture with acetone, methyl ethyl ketone, acetonitrile or tetrahydrofuran or mixtures thereof.

In a more preferred embodiment, acetone or tetrahydrofuran or mixtures thereof or water in admixture with acetone or tetrahydrofuran or mixtures thereof are used.

In a further more preferred embodiment, tetrahydrofuran or water in admixture with tetrahydrofuran can be used.

In a most preferred embodiment, acetone or water in admixture with acetone can be used.

Preferably, an admixture with water is used in order to provide a sufficiently high solubility which is needed for filtration of the reaction mixture. On the other hand the amount of water should be as low as possible to achieve high yields. Preferred ratios of water/acetone are 1 : 0.9 (m/m) to 1 : 1 (m/m). However the reaction itself can in principle be conducted in any ratio of water/acetone.

Hydrochloric acid is as a rule applied as concentrated hydrochloric acid with a HCl content of about 37 % in water.

The deprotection reaction can expediently be performed a temperature between 30 °C and 80 °C, depending on the solvent. Preferably, a temperature in the range of 35 °C to 66 °C is applied.

The desired product of formula I can be as a rule isolated by diluting the mixture with acetone and subsequent filtration followed by washing with the solvent in the form of colorless crystals. Preferably, the product is obtained in yields of ≥ 90% and having an assay of ≥ 98%.

Description of the figures:
Figure 1: shows a XRPD (Powder X-Ray Diffraction) pattern of a typical lot of form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 2: shows an IR (Infra Red spectroscopy) spectrum of a typical lot of form A of (2S,3S,11bs)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 3: shows a DSC (Differencial Scanning Calorimetry) curve of a typical lot of form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figures 4: shows a TGA (Thermo Gravimetric Analysis) curve of a typical lot of form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 5: shows a XRPD (Powder X-Ray Diffraction) pattern of a typical lot of form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figures 6: shows an IR (Infra Red spectroscopy) spectrum of a typical lot of form B of (2S,3S, 11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 7: shows a DSC (Differencial Scanning Calorimetry) curve of a typical lot of form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 8: shows a TGA (Thermo Gravimetric Analysis) curve of a typical lot of form B of (2S,3S,11bS)-1-(2-amino-9,10-dimethoxy-1,3,4,6,7,11b-hexahydro-2H-pyrido [2,1-a]isoquinolin-3-yl)-4(S)-fluoromethyl-pyrrolidin-2-one dihydrochloride.
Figure 9: shows a XRPD (Powder X-Ray Diffraction) pattern of a typical lot of the amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 10: shows an IR (Infra Red spectroscopy) spectrum of a typical lot of the amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 11: shows a DSC (Differencial Scanning Calorimetry) curve of a typical lots of the amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 12: shows a TGA (Thermo Gravimetric Analysis) curve of a typical lot of the amorphous form of (2S,3S, 11bS)-1-(2-amino-9, 10-dimethoxy-1,3,4,6,7,11b-hexahydro-2H-pyrido[2,1-a]isoquinolin-3-yl)-4(S)-fluoromethyl-pyrrolidin-2-one dihydrochloride.
Figure 13: shows a thermal ellipsoid plot of the crystal structure of form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.

The term "polymorph" refers to a crystal form or modification which can be characterized by analytical methods such as e.g. X-ray powder diffraction, IR spectroscopy or differencial scanning calorimetry.

As used herein, "amorphous form" denotes a material that lacks long range order and as such does not show sharp X-ray peaks. The XRPD pattern of an amorphous material is characterized by one or more amorphous halos.

"DMF" is used herein as an acronym of N,N-Dimethylformamide.

"DSC" is used herein as an acronym of Differencial Scanning Calorimetry. DSC curves were recorded using a Mettler-Toledo™ differential scanning calorimeter DSC820 or DSC 821 with a FRS05 sensor. System suitability tests were performed with indium as reference substance and calibrations were carried out using indium, benzoic acid, biphenyl and zinc as reference substances.

For the measurements approximately 2 to 6 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 100 mL/min using heating rates of usually 10 K/min.

"Form A" is used herein as abbreviation for the crystalline form A of (2S,3S,11bS)-1-(2-amino-9,10-dimethoxy-1,3,4,6,7,11b-hexahydro-2H-pyrido[2,1-a] isoquinolin-3-yl)-4(S)-fluoromethyl-pyrrolidin-2-one dihydrochloride.

"Form B" is used herein as abbreviation for the crystalline DMF solvate form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.

"IR" is used herein as an acronym of Infra Red spectroscopy. The IR-spectrum of the sample was recorded as film of a Nujol suspension consisting of approx. 5 mg of sample and few Nujol between two sodium chloride plates, with an FT-IR spectrometer in transmittance. The Spectrometer was a Nicolet™ 20SXB or equivalent (resolution: 2 cm, 32 or more coadded scans, MCT detector).

"XRPD" is used herein as an acronym of X-Ray Powder Diffraction. X-ray diffraction patterns were recorded in transmission geometry with a STOE STADIP diffractometer with CuKα radiation (1.54 Å) and a position sensitive detector. The samples (approximately 50 mg) were prepared between thin polymer (or aluminum) films and analyzed without further processing (e.g. grinding or sieving) of the substance.

Alternatively, X-ray diffraction patterns were measured on a Scintag X1 powder X-ray diffractometer equipped with a sealed copper Kα1 radiation source. The samples were scanned from 2° to 36° 2θ at a rate of 1° per minute with incident beam slit widths of 2 and 4 mm and diffracted beam slit widths of 0.3 and 0.2mm.

For single crystal structure analysis a single crystal was mounted in a loop on a goniometer and measured at ambient conditions. Alternatively, the crystal was cooled in a nitrogen stream during measurement. Data were collected on a STOE Imaging Plate Diffraction System (IPDS) from STOE (Darmstadt). In this case Mo-radiation of 0.71 Å wavelength was used for data collection. Data was processed with STOE IPDS-software. The crystal structure was solved and refined with standard crystallographic software. In this case the program ShelXTL from Bruker AXS (Karlsruhe) was used.

Alternatively, synchrotron radiation was used for data collection. A single crystal was mounted in a loop and cooled to 89 K in a nitrogen stream. Data was collected at the Swiss Light Source beamline X10SA using a MAR CCD225 detector with synchrotron radiation (0.80 Å) and data processed with the program XDS. The crystal structure was solved and refined with standard crystallographic software. In this case the program ShelXTL from Bruker AXS (Karlsruhe) was used.

"TGA" is used herein as an acronym of Thermo Gravimetric Analysis. TGA analysis was performed on a Mettler-Toledo^{™} thermogravimetric analyzer (TGA850 or TGA851). System suitability tests were performed with hydranal as reference substance and calibrations were carried using aluminium and indium as reference substances.

For the thermogravimetric analyses, approx. 5 to 10 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 50 mL/min using a heating rate of 5 K/min.

It has been found that (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride (VII) can be isolated, depending upon the method of preparation, as form A, B or in an amorphous form. Form A can be isolated from different crystallization methods as described below. Form B can be isolated from crystallization in DMF or DMF/water. The amorphous form can be obtained by lyophilization of an aqueous solution of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.

Form A can be obtained by recrystallization of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride in a mixture of methanol/water (0.5:0.5 w/w) at certain temperature and concentration after seeding with subsequent precipitation during cooling. Form A can also be obtained by recrystallization of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride in a solvent selected from the group consisting of methanol, ethanol, ethanol/water mixtures, acetone/water mixtures, tetrahydrofurane, 2-propanol and acetonitrile and spontaneous crystallization, without seeding, with subsequent precipitation during cooling.

Form A of (2S,3S,11HbS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride is a solvent-free form as no significant weight loss is observed in the TGA curve prior to decomposition and can be characterized:
- by the following X-ray diffraction pattern obtained with a CuK_{α} radiation having characteristic peaks expressed in degrees 2Theta at approximately: 6.0, 10.1, 12.1, 13.2, 14.5, 15.2, 15.5, 16.1, 16.4, 17.7, 19.5, 20.7, 21.6, 22.6, 27.3, 27.8 and 30.2. The term "approximately" means in this context that there is uncertainty in the measurements of the degrees 2Theta of ± 0.2 (expressed in degrees 2Theta);
- by an IR absorption spectrum having characteristic peaks expressed in cm⁻¹ at approximately 3582 cm⁻¹, 3440 cm⁻¹, 3237 cm⁻¹, 2726 cm⁻¹, 2535 cm⁻¹, 2477 cm⁻¹, 1953 cm⁻¹, 1681 cm⁻¹, 1601 cm⁻¹, 1575 cm⁻¹, 1525 cm⁻¹, 1491 cm⁻¹, 1408 cm⁻¹, 1308 cm⁻¹, 1260 cm⁻¹, 1225 cm⁻¹, 1193 cm⁻¹, 1145 cm⁻¹, 1130 cm⁻¹, 1096 cm⁻¹, 1054 cm⁻¹, 1000 cm⁻¹, 967 cm⁻¹, 946 cm⁻¹, 879 cm⁻¹, 844 cm⁻¹, 808 cm⁻¹, 768cm⁻¹ and 654 cm⁻¹. The term "approximately" means in this context that the cm⁻¹ values can vary, e.g. by up to ± 3 cm⁻¹; and
- by a melting range (DSC) of about 295 °C to 310 °C under severe decomposition.

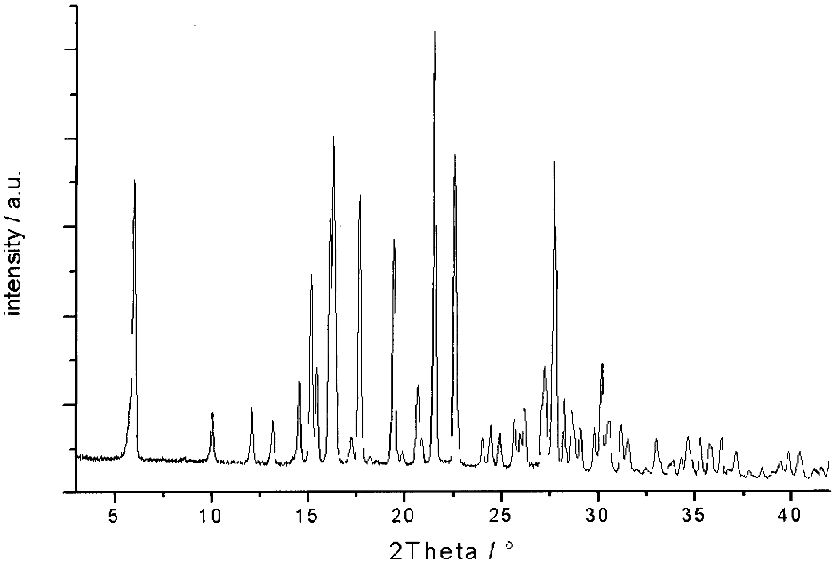

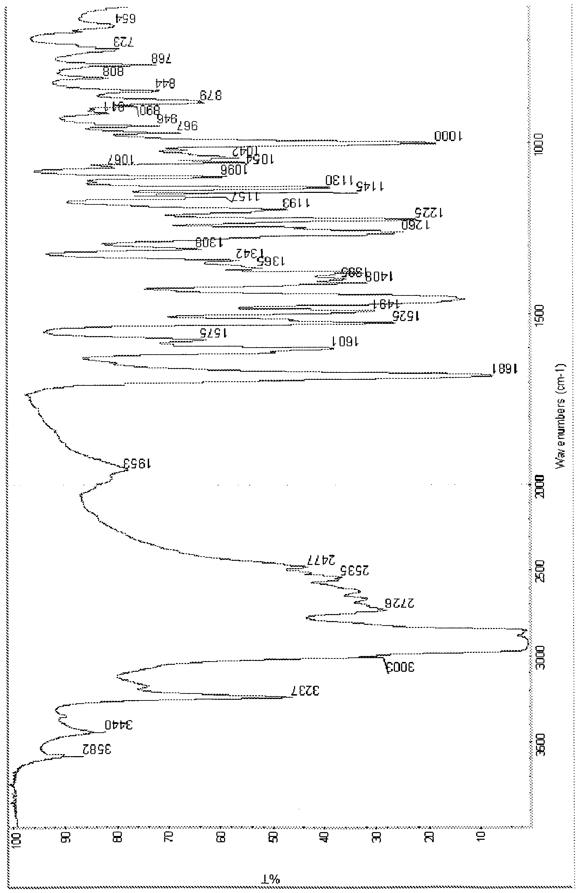

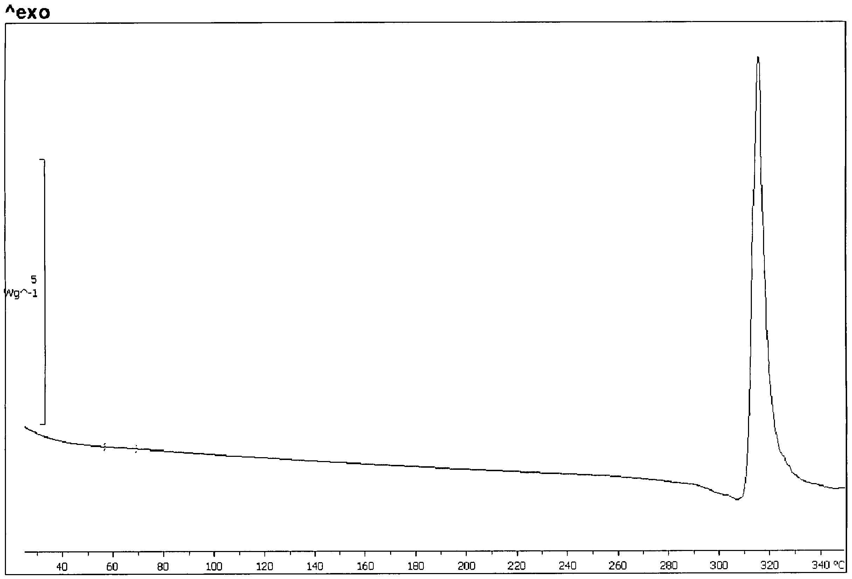

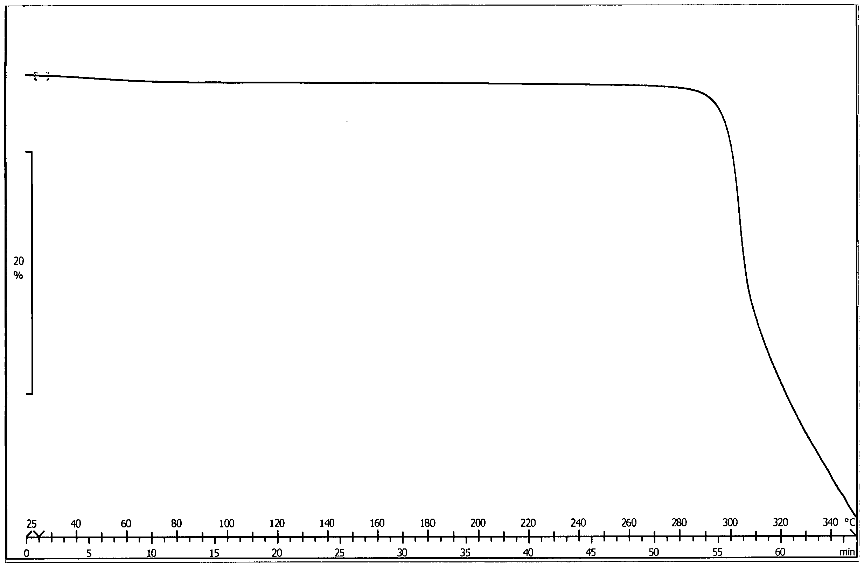

These characteristics and others are shown in figures 1 to 4.

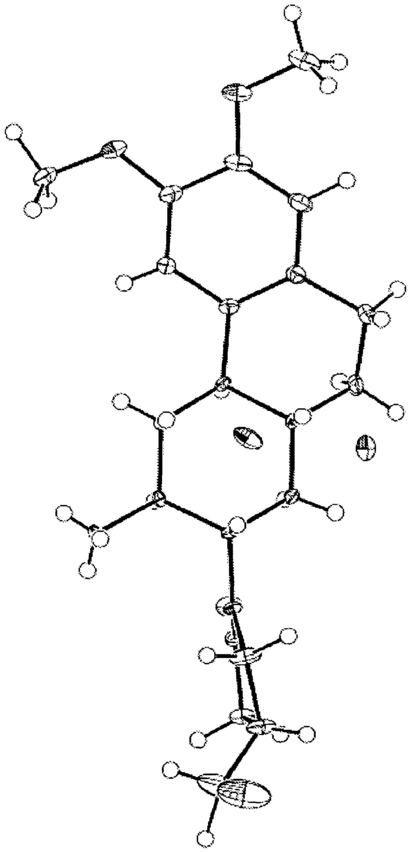

A single crystal structure analysis of form A was conducted. Table 1 lists some crystal structure data. The experimental X-ray powder diffraction pattern of form A corresponds to the theoretical pattern calculated from crystal structure data collected at ambient conditions. The crystal packing of form A shows hydrogen bonds of the carbonyl oxygen with the protonated primary amino groups of two adjacent active molecules. A thermal ellipsoid plot of the crystal structure is shown in figure 13.

**Table 1: Crystal structure data of form A**

| | | |
|---|---|---|
| form | --- | A |
| crystal system | --- | Orthorhombic |
| space group | --- | P 2(1) 2(1) 2(1) |
| crystal habit | --- | platelet |
| | [Å] | a = 6.77 |
| unit cell dimensions | [Å] | b = 10.98 |
| | [Å] | c = 29.04 |
| | [°] | α, β, γ = 90.0 |
| temperature | [K] | 89 |
| cell volume | [Å³] | 2159 |
| molecules in unit cell | --- | 4 |
| calculated density | [g/cm³] | 1.39 |

Form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride, a DMF solvate, can be obtained by stirring a DMF/water (0.5:0.5 w/w) suspension of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride several days.

Form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride is a solvate form and a weight loss 5.4 % - 5.7 % and 11.8 % - 13.2% at 25 °C up to approximately 150 °C respective approximately 150 °C up to 190 °C is observed in the TGA curve prior to decomposition and can be characterized:
- by the following X-ray diffraction pattern obtained with a CuK_{α} radiation radiation having characteristic peaks expressed in degrees 2Theta at approximately: 6.8, 12.5, 13.5, 15.1, 17.4, 18.1, 18.4, 24.3, 24.8, 25.3, 27.2, 27.9, 28.1, 29.9 and 30.7. The term "approximately" means in this context that there is an uncertainty in the measurements of the degrees 2Theta of ± 0.2 (expressed in degrees 2Theta).
- by an IR absorption spectrum having characteristic peaks expressed in cm⁻¹ at approximately 3480 cm⁻¹, 3376 cm⁻¹, 2706 cm⁻¹, 2682 cm⁻¹, 2610 cm⁻¹, 2574 cm⁻¹, 2532 cm⁻¹, 2381 cm⁻¹, 1684 cm⁻¹, 1659 cm⁻¹, 1622 cm⁻¹, 1574 cm⁻¹, 1528 cm⁻¹, 1487 cm⁻¹, 1410 cm⁻¹, 1383 cm⁻¹, 1310 cm⁻¹, 1267 cm⁻¹, 1251 cm⁻¹, 1229 cm⁻¹, 1192 cm⁻¹, 1135 cm⁻¹, 1107 cm⁻¹, 998 cm⁻¹, 988 cm⁻¹, 930 cm⁻¹, 900 cm⁻¹, 841 cm⁻¹, 767 cm⁻¹, 680 cm⁻¹, and 667 cm⁻¹. The term "approximately" means in this context that the cm⁻¹ values can vary, e.g. by up to ± 3 cm⁻¹.
- by an endothermic event at 171 °C to 175 °C (extrapolated Peak; DSC).

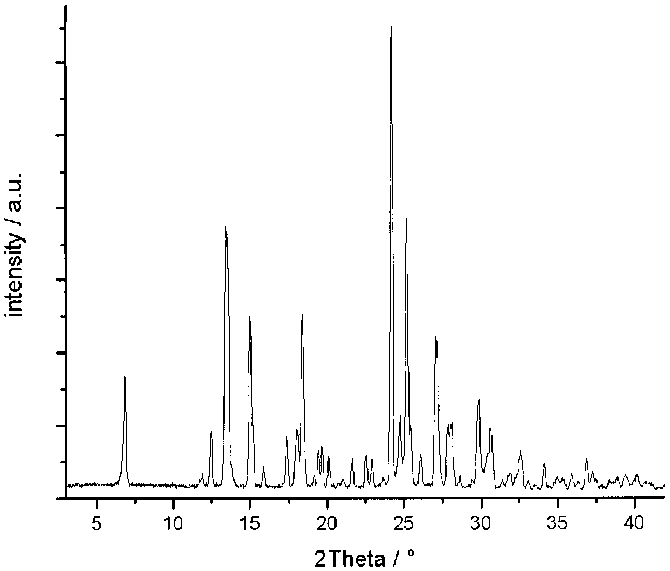

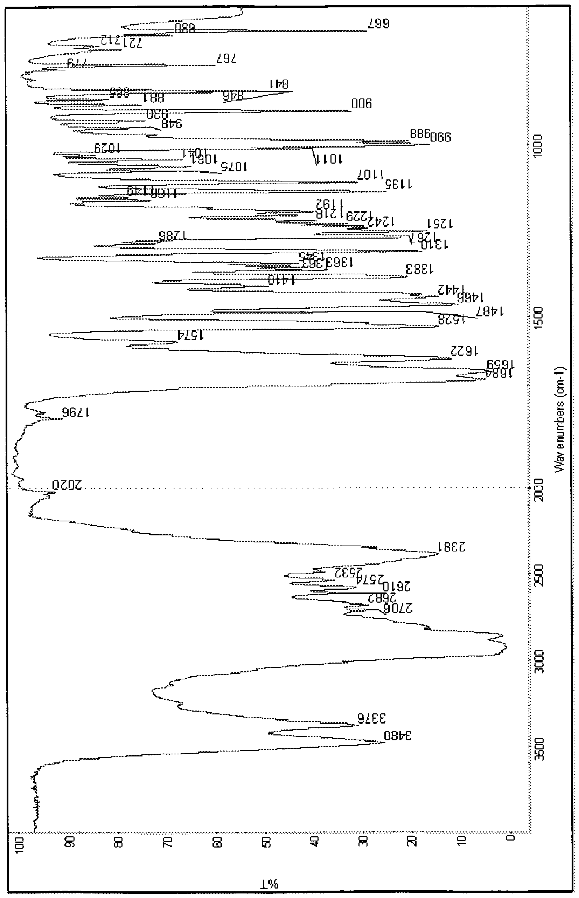

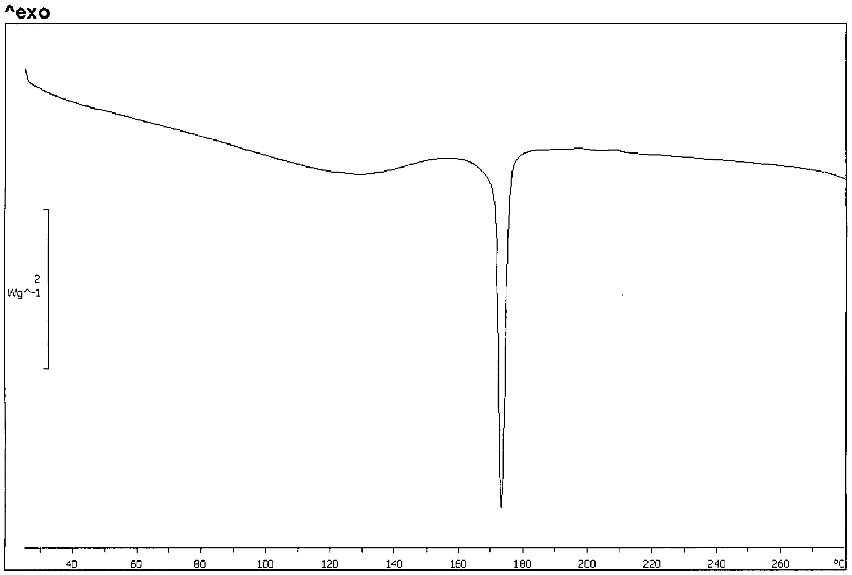

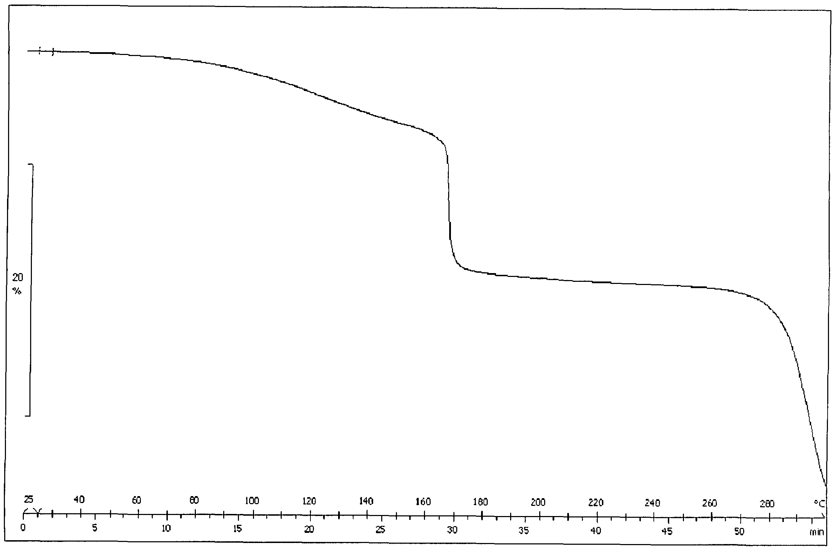

These characteristics and others are shown in figures 5 to 8.

The amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride can be obtained by lyophilisation of a solution of 5.0 g of (2S,3S,11bS)-1-(2-amino-9,10-dimethoxy-1,3,4,6,7,11b-hexahydro-2H-pyrido [2,1-a] isoquinolin-3-yl)-4(S)-fluoromethyl-pyrrolidin-2-one dihydrochloride in 20 mL water (condensator at -40 °C and vacuum at 0 to 1 mbar)

The amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride can be characterized:
- by the lack of sharp X-ray diffraction peaks in its XRPD pattern;
- by an IR absorption spectrum having characteristic peaks expressed in cm⁻¹ at approximately 3429 cm⁻¹, 2507 cm⁻¹, 1680 cm⁻¹, 1612 cm⁻¹, 1515 cm⁻¹, 1310 cm⁻¹, 1261 cm⁻¹, 1246 cm⁻¹, 1219 cm⁻¹, 1127 cm⁻¹, 994 cm⁻¹, 964 cm⁻¹, 945 cm⁻¹, 888 cm⁻¹, 860 cm⁻¹, 842 cm⁻¹, 767 cm⁻¹, 685 cm⁻¹ and 635 cm⁻¹. The term "approximately" means in this context that the cm⁻¹ values can vary, e.g. by up to ± 3 cm⁻¹.

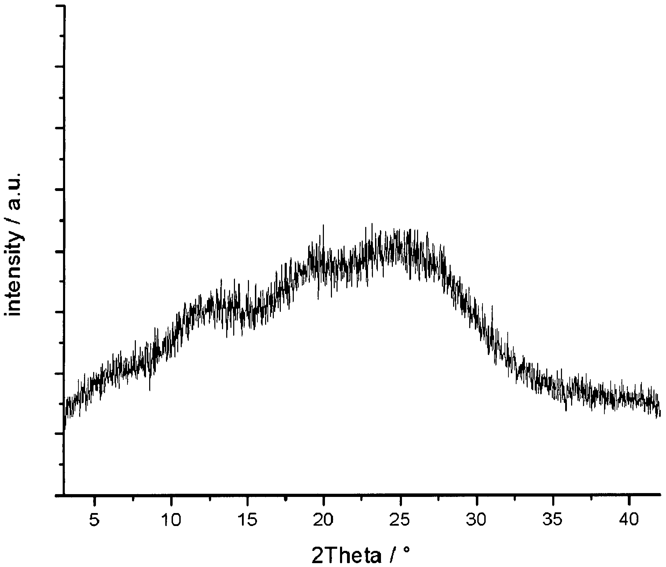

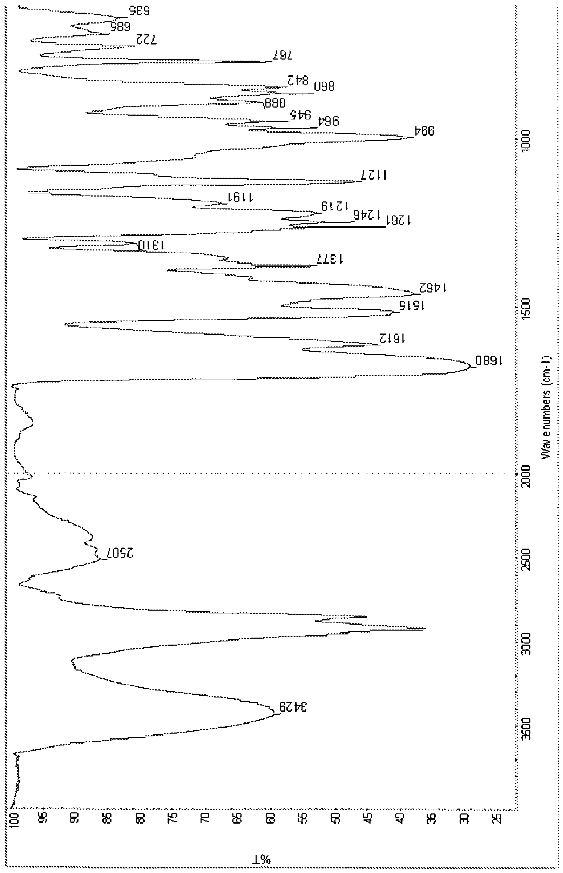

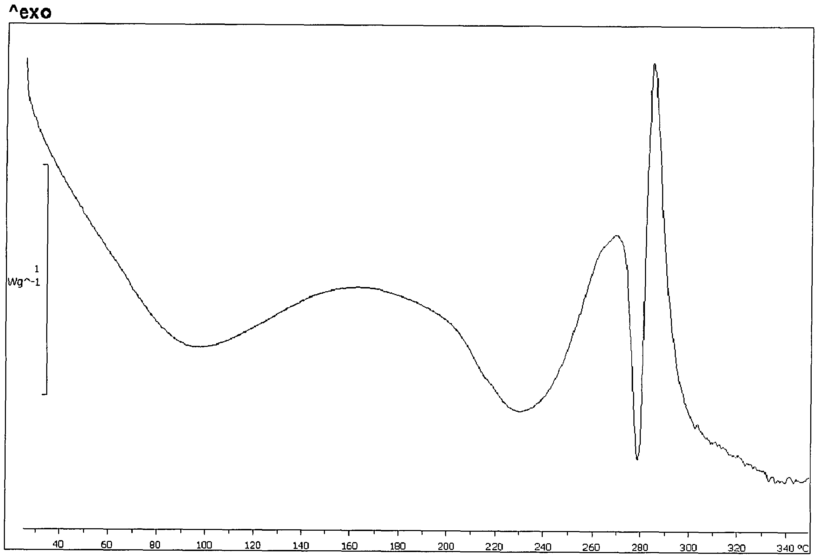

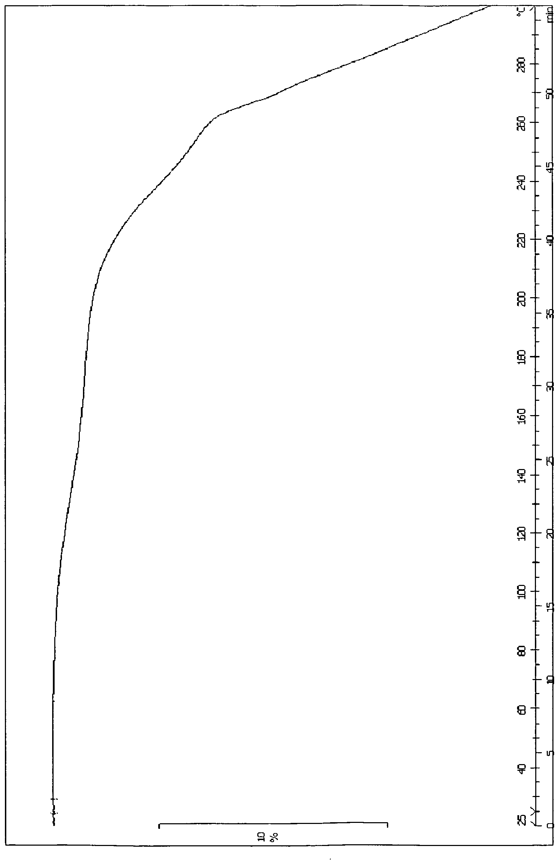

These characteristics and others are shown on figures 9 to 12.

### Examples

### Abbreviations

| | |
|---|---|
| MEON | Methanol |
| EtOH | Ethanol |
| THF | Tetrahydrofuran |
| ACN | Acetonitrile |
| IPA | 2-Propanol |
| MEK | Methyl ethyl ketone |
| RT | Room Temperature |

### Determination of the assays

The assay of (2*S*,3*S*,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2) was determined by HPLC analysis, using a XBridge C18 column of Waters, 3,5 µm, 4.6 x 150 mm , UV detection at 284 nm, gradient with mixtures of water, acetonitrile and trietylamine, flow rate of 1 mL/min and column oven temperature adjusted to 40 °C and employing an external standard.

The assay of (2*S*,3*S*,11*bS*)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride (3) was determined by HPLC analysis, using a Atlantis T3 column of Waters, 3 µm, 4.6 x 150 mm, UV detection at 284 nm, gradient with mixtures of water, acetonitrile and potassium dihydrogen phosphate buffer and pH of 3.0, flow rate of 1.0 mL/min and column oven temperature adjusted to 45 °C and employing an external standard.

### Example 1

Preparation of (2*S*,3*S*,11*bS*)-3-((4*S*)-Fluoromethyl-2-oxo-pyrrolidin-1-yl)-9, 10-dimethoxy-1,3,4,6,7,11b-hexahydro-2H-pyrido[2,1-a]isoquinolin-2-yl]-carbamic acid tert-butyl ester (2)

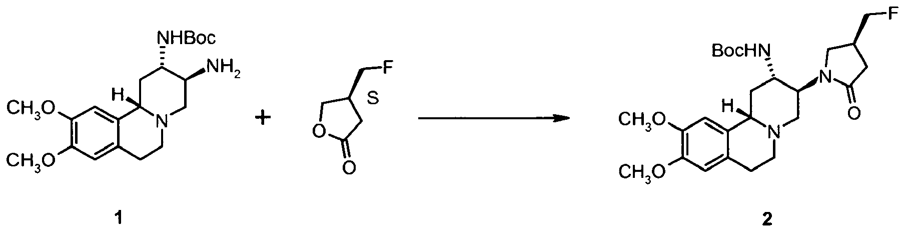

### Example 1a (with LHMDS as base for cyclization)

A 1.2 L reactor equipped with a mechanical stirrer, a Pt-100 thermometer, a dropping funnel and a nitrogen inlet was charged with 30.0 g (79.5 mmol) of (2S,3S,11bS)-(3-amino-9,10-dimethoxy-1,3,4,6,7,1b-hexahydro-2H-pyrido[2,1-a] isoquinolin-2-yl)]-carbamic acid tert-butyl ester (1) and 1.20 g (9.1 mmol) 6-chloro-2-pyridinol in 450 ml toluene. The mixture was heated to 85 to 90 °C and 12.2 g (103 mmol) of (S)-4-fluoromethyl-dihydro-furan-2-one was added within 45-60 minutes. After the addition, the mixture was heated to 100 to 110 °C and stirred at this temperature for 8 hours. Around 200 ml of toluene were then distilled off and the resulting thick suspension was stirred at 85 °C for another 10 to 15 hours. The mixture was then allowed to cool to 25 to 30 °C and 450 ml of THF were added. The mixture was then treated at 25 to 30 °C with 12.9 g (111 mmol) methanesulfonyl chloride followed by 13.1 g (130 mmol) of triethylamine. The resulting thick suspension was allowed to stir at 25 to 30 °C for 60 to 90 minutes, then cooled to -10 to 0 °C and treated at this temperature within 1 to 2 hours with 168 g (238 mmol) lithium-bis (trimethylsilyl) amide (23.8% in THF). After complete addition, the almost clear solution was stirred for additional 1 to 2 hours at -10 to 0 °C. The mixture was then quenched with 75 ml of water. The layers were separated and the organic layer was washed with water (1 × 75 ml). From the organic layer THF and toluene were completely distilled off and replaced by MeOH. The resulting suspension (approx. 250 ml) was heated to reflux temperature and then allowed to cool to -20 °C within 4 to 6 hours. The resulting suspension was stirred for 2 hours at -20 °C. The crystals were filtered off, washed with 60 ml of pre-cold MeOH and dried at 45 °C / <30 mbar to afford 29.8 g of the title product as colorless crystals (77% yield; assay: 97.5 %(m/m)).

### Example 1b (with LiOtBu as base for cyclization)

A 1.2 L reactor equipped with a mechanical stirrer, a Pt-100 thermometer, a dropping funnel and a nitrogen inlet was charged with 30.0 g (79.5 mmol) of (2S,3S,11bS)-(3-amino-9,10-dimethoxy-1,3,4,6,7,11b-hexahydro-2 H-pyrido[2,1-a] isoquinolin-2-yl)]-carbamic acid tert-butyl ester (1) and 1.20 g (9.1 mmol) 6-chloro-2-pyridinol in 450 ml toluene. The mixture was heated to 85 to 90 °C and 12.2 g (103 mmol) of (S)-4-fluoromethyl-dihydro-furan-2-one was added within 45-60 minutes. After the addition, the mixture was heated to 105 °C and stirred at this temperature for 5 hours. Approx. 250 ml of toluene were then distilled off and the resulting thick suspension was stirred at 85 °C for another 16 hours. 100 ml of toluene were then distilled off and replaced by 400 g of THF. At the end of the distillation a reaction volume of 500 ml was adjusted. The mixture was then cooled to 23°C and treated at 23-30 °C with 13.8 g (0.12 mol) methanesulfonyl chloride followed by 14.0 g (0.14 mol) of triethylamine. The resulting thick suspension was allowed to stir at 25 to 30 °C for 75 minutes, then cooled to -10 to 0 °C and treated at this temperature within 1 to 2 hours with 95 g (0.24 mol) lithium-tert.-butoxide (20% in THF). After addition completion, the suspension was stirred for 2 hours at -5 °C. The mixture was then quenched with 75 g of water. The layers were separated and the organic layer was washed with 70 g sulfuric acid solution (2.5% in water). From the organic layer THF and toluene were completely distilled off and replaced by MeOH. The resulting suspension (approx. 230 ml) was heated to reflux temperature and then allowed to cool to -10 °C within 5 hours. The suspension was stirred for 2 hours at -10 °C. The crystals were filtered off, washed with 50 ml of pre-cold MeOH and dried at 45 °C / <30 mbar to afford 30.02 g of the title product as colorless crystals (79% yield; assay: 99.2 %(m/m)).

### Example 2

Preparation of (2*S*,3*S*,11*bS*)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride (VII)

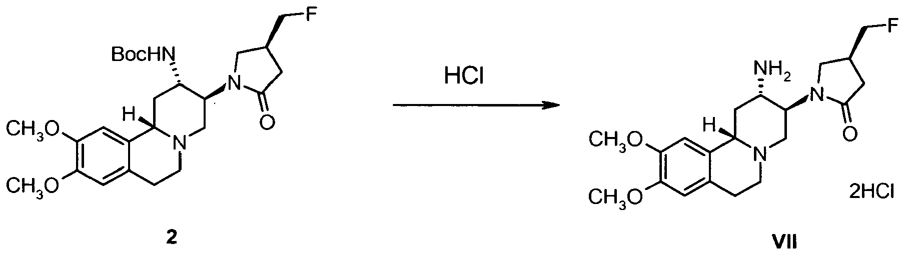

### Example 2a (in THF / water as the solvent)

A 350 ml reactor equipped with a mechanical stirrer, a Pt-100 thermometer, a dropping funnel and a nitrogen inlet was charged with 15.0g (30.8 mmol) of (2*S*,3*S*,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 120 ml of THF and 3 ml of water. At a temperature between 20 to 30 °C 9.15 g (~ 93 mmol) of hydrochloric acid (37% in water) were added within 15 to 30 minutes. The resulting solution was heated for 4 hours at 45 °C. The suspension cooled down to 10 to 15 °C and then stirred at this temperature for 2 to 3 hours. The crystals were filtered off, washed with 40 ml of THF and dried at 45 °C / <30 mbar to afford 13.5 g of the title compound as colorless crystals (96% yield; assay: 98.9 %(m/m)).

### Example 2b (in acetone / water as the solvent)

A 250 ml double jacket reactor equipped with a mechanical stirrer, a Pt-100 thermometer, a dropping funnel and a nitrogen inlet was charged with 21.10g (43.74 mmol) of (2*S*,3*S*,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g acetone and 19.0 g of water. At a temperature between 47 to 53 °C 15.2 g (153 mmol) hydrochloric acid (37% in water) were added within 10 minutes. The obtained solution was filter through a 5 µm sieve and the 250 ml reactor was washed with a mixture of 18.0 g acetone and 2.0 g water. The filtered solution was transferred into a 500 ml double jacket reactor equipped with an agitator, Pt-100 thermometer, a dropping funnel and a nitrogen inlet. Under stirring at 20 to 30 °C 250 g of acetone were added to the solution within 1-2 hours. Subsequently the mixture was stirred for additional 2 hours at 20 to 25 °C. The crystals were filtered of, washed with 200 g of acetone and dried at 90 °C / <20 mbar to afford 19.70 g of the title compound as colorless crystals (92.3% yield; assay: 99.9 %(m/m)).

### Example 2c (in acetone / water as the solvent)

A 250 ml double jacket reactor equipped with a mechanical stirrer, a Pt-100 thermometer, a dropping funnel and a nitrogen inlet was charged with 21.10g (43.74 mmol) of (2S,3S,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g Acetone and 19.0 g of water. At a temperature between 47 to 53 °C 15.2 g (153 mmol) hydrochloric acid (37% in water) were added within 10 minutes. The obtained solution was filter through a 5 µm sieve and the 250 ml reactor was washed with a mixture of 18.0 g Acetone and 2.0 g water. The filtered solution was transferred into a 500 ml double jacket reactor equipped with an agitator, Pt-100 thermometer, a dropping funnel and a nitrogen inlet. Under stirring at 38 to 42 °C 250 g of ACN were added to the solution within 1-2 hours. Subsequently the mixture was stirred for additional 2 hours at 38 to 42 °C. The crystals were filtered of, washed with 80 g of ACN and dried at 90 °C / <20 mbar to afford 19.80 g of the title compound as colorless crystals (93.5% yield; assay: 98.8 %(m/m)).

### Example 2d (in MEK/ water as the solvent)

A 250 ml double jacket reactor equipped with a mechanical stirrer, a Pt-100 thermometer, a dropping funnel and a nitrogen inlet was charged with 21.10g (43.74 mmol) of (2*S*,3*S*,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g MEK and 18.0 g of water. At a temperature between 47 to 53 °C 15.2 g (153 mmol) hydrochloric acid (37% in water) were added within 10 minutes. The obtained solution was filter through a 5 µm sieve. The filtered solution was transferred into a 500 ml double jacket reactor equipped with an agitator, Pt-100 thermometer, a dropping funnel and a nitrogen inlet. Under stirring at 20 to 25 °C 80 g of MEK were added to the solution within 1 to 2 hours. Subsequently the mixture was stirred for additional 16 hours at 20 to 22 °C. 120g Acetone were added within 10 minutes. After additional stirring at 20 to 22°C for 4 to 5 hours the crystals were filtered of, washed with 200 g of Acetone and dried at 90 °C / <20 mbar to afford 16.96 g of the title compound as colorless crystals (86.1% yield; assay: 99.1 %(m/m)).

In the table below examples with different solvents are provided to illustrate the invention.

| Example | Solvent composition for the reaction (debocylation)* | Solvent composition for crystallisation/isolation* | Yield [%] | Assay [%] |
|---|---|---|---|---|
| 2e | Water | Water / ACN 1 / 13.95 | 71,5 | 99,6 |
| 2f | ACN / water 1 / 0.875 | ACN / water / acetone 1 / 0.64 / 17.24 | 87,9 | 99,0 |
| 2c | Water / acetone 1 / 0.95 | Water / acetone / ACN 1 / 0.55 / 6.58 | 93,5 | 98,8 |
| 2d | MEK/ water 1/0.9 | Methyl ethyl ketone/ water / acetone 1 / 0.18 / 1.2 | 86,1 | 99,1 |

| | | | | |
|---|---|---|---|---|
| * In m/m. The water of the hydrochloric acid (37% in water) is not included. | | | | |

### Comparison example 2A (in MeOH as the solvent)

A 350 ml reactor equipped with a mechanical stirrer, a Pt-100 thermometer, a dropping funnel and a nitrogen inlet was charged with 10.0g (20.7 mmol) of (2*S*,3*S*,11*bS*)-3-((4*S*)-Fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2) and 50 ml of MeOH. The suspension was heated to 50 °C and treated at this temperature within 5 minutes with 10.2 g hydrochloric acid (37% in water). The mixture was stirred for 4 hours at 50 °C. The colorless suspension was then allowed to cool to RT and then treated within 15 to 30 minutes with 100 ml of methyl acetate. The suspension was cooled to 0 to 5°C and stirred at this temperature for 1 to 2 hours. The crystals were filtered off, washed with a mixture of methyl acetate and methanol and dried at 70 °C / <30 mbar to afford 8.89 g of the title compound as colorless crystals (93% yield; assay: 98%(m/m)) with a methyl chloride content of several hundred ppm.

### Comparison example 2B (in MeOH/water as the solvent)

A 250 ml double jacket reactor equipped with a mechanical stirrer, a Pt-100 thermometer, a dropping funnel and a nitrogen inlet was charged with 21.10g (43.74 mmol) of (2*S*,3*S*,11*bS*)-3-((4*S*)-fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g methanol and 15.0 g of water. At a temperature between 47 to 53 °C 15.2 g (153 mmol) hydrochloric acid (37% in water) were added within 10 minutes. The obtained solution was filter through a 5 µm sieve. The filtered solution was transferred into a 500 ml double jacket reactor equipped with an agitator, Pt-100 thermometer, a dropping funnel and a nitrogen inlet. Under stirring at 20 to 30 °C 250 g of Acetone were added to the solution within 1 to 2 hours. After seeding with 200 mg of VII the mixture started crystallization. Subsequently the mixture was cooled down to -5 to 0 °C and was stirred for additional 16 hours. The crystals were filtered of, washed with 40 g of Acetone and dried at 90 °C / <20 mbar to afford 19.44 g of the title compound as colorless crystals (91.8% yield; assay: 98.5 %(m/m)) with a methyl chloride content between 100 and 200 ppm.

### Comparison Example 2C (in IPA/water as the solvent)

A 250 ml double jacket reactor equipped with a mechanical stirrer, a Pt-100 thermometer, a dropping funnel and a nitrogen inlet was charged with 21.10g (43.74 mmol) of (2*S*,3*S*,11*bS*)-3-((4*S*)-fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g IPA and 18.0 g of water. At a temperature between 47 to 53 °C 15.2 g (153 mmol) hydrochloric acid (37% in water) were added within 10 minutes. The obtained solution was filter through a 5 µm sieve. The filtered solution was transferred into a 500 ml double jacket reactor equipped with an agitator, Pt-100 thermometer, a dropping funnel and a nitrogen inlet. Under stirring at 20 to 25 °C 250 g of IPA were added to the solution within 1 to 2 hours. After seeding with 200 mg of 3 the mixture started crystallization. Subsequently the mixture was stirred for additional 3 hours at 20 to 22 °C. The crystals were filtered of, washed with 40 g of IPA and dried at 90 °C / <20 mbar to afford 18.22 g of the title compound as colorless crystals (92.50 % yield; assay: 98.4 %(m/m)) with a isopropyl chloride content of approximately 100 ppm.

### Comparison Example 2D (in EtOH / water as the solvent)

A 250 ml double jacket reactor equipped with a mechanical stirrer, a Pt-100 thermometer, a dropping funnel and a nitrogen inlet was charged with 21.10 g (43.74 mmol) of (2*S*,3*S*,11*bS*)-3-((4*S*)-fluoromethyl-2-oxo-pyrrolidin-1-yl)-9,10-dimethoxy-1,3,4,6,7,11b-hexahydro-2H-pyrido[2,1-a]isoquinolin-2-yl]-carbamic acid tert-butyl ester (2), 20.0 g EtOH and 18.0 g of water. At a temperature between 47 to 53 °C 15.2 g (153 mmol) hydrochloric acid (37% in water) were added within 10 minutes. The obtained solution was filter through a 5 µm sieve. The filtered solution was transferred into a 500 ml double jacket reactor equipped with an agitator, Pt-100 thermometer, a dropping funnel and a nitrogen inlet. Under stirring at 20 to 25 °C 250 g of ethanol were added to the solution within 1 to 2 hours. After seeding with 200 mg of 3 the mixture started crystallization. Subsequently the mixture was stirred for additional 3 hours at 20 to 22 °C. The crystals were filtered of, washed with 40 g of Ethanol and dried at 90 °C / <20 mbar to afford 19.44 g of the title compound as colorless crystals (91.8% yield; assay: 98.4 %(m/m)) with a ethyl chloride content between 200 and 300 ppm.

### Example 3

Preparation of form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride

Form A of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride can be produced by digestion in solvents as e.g. methanol, ethanol, 2-propanol or solvent mixtures as acetone/water (e.g. 1:1, w/w), water/methanol (e.g. 1:1, w/w), water/ethanol (e.g. 0.4:0.6 w/w). It can also be prepared by re-crystallization of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride with or without seeding in solvent systems comprising but not limited to methanol, methanol/water (e.g. 0.5:0.5, w/w), ethanol, water/ethanol (e.g. 0.6:0.4, w/w).

### Crystallization Procedure

250.00 g of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride were dissolved in 91.2 g acetone and 91.2 g water and heated up to 50 to 55 °C. The solution was hot filtered. To the clear solution at 50 to 55 °C 4763.0 g of acetone were added. After the addition of 400 mL of acetone 2.0 g of form A seeding crystals of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride were added at 50 to 55 °C. After complete addition of the acetone the temperature of the suspension was cooled with 0.3 to 0.6 K/min down to 5 to 10 °C. After stirring at 5 to 10 °C for 1 h the colorless crystals were filtered, washed with ca. 200 mL of acetone (5 to 10 °C) and dried at 70 °C / < 20 mbar for 14 h. Yield: 241.0 g (95.4 %).

### Preparation of seeding crystals of form A

Form A seeding crystals can be prepared by digestion of a slurry of (2S,3S,1 11bS) -1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride in solvent systems comprising but not limited to ethanol, methanol and water mixtures of ethanol/water (e.g. 0.4:0.6 w/w). After stirring the slurry at room temperature for several days form A crystals can be filtered and were dried at 70 °C / < 20 mbar for 14 h.

### Solid state properties of form A

XRPD-pattern, IR-spectrum, DSC curve, and TG curve of form A are shown in Figures 1 to 4.

### Example 4

Preparation of form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride

Form B of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride, a DMF solvate can be prepared by digestion of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride in the solvent system DMF/water (e.g. 0.5:0.5 w/w).

### Crystallization Procedure

0.38 g of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride were suspended in 0.50 g DMF/water (0.5:0.5 w/w) and stirred for five days at ambient temperature. After filtration and drying at 40 °C to 50 °C / <20 mbar for several hours 0.04 g of a white solid were isolated. Yield: 0.04 g.

### Solid state properties of form B

XRPD-pattern, IR-spectrum, DSC curve, and TG curve of form B are shown in Figures 5 to 8.

### Example 5

Preparation of the amorphous form of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride

An amorphous form of (2S,3S,11bS)-1-(2-amino-9,10-dimethoxy-1,3,4,6,7,11b-hexahydro-2H-pyrido[2,1-a] isoquinolin-3-yl)-4(S)-fluoromethyl-pyrrolidin-2-one dihydrochloride was accessible by lyophilisation.

### Preparation Procedure

5.00 g of (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride were dissolved in 20 g of water at ambient temperature. The clear solution was frozen and lyophilized under reduced pressure (condensator at -55 °C and vacuum at 0.2 mbar) for 72 hours. Analysis revealed amorphous (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride. Yield: 4.7 g (94 %).

### Solid state properties of the amorphous form

XRPD-pattern, IR-spectrum, DSC curve and TG curve of the amorphous form are shown in Figures 9 to 12.

## Claims

1. Process for the preparation of a pyrido[2,1-a]isoquinoline derivative of formula
I 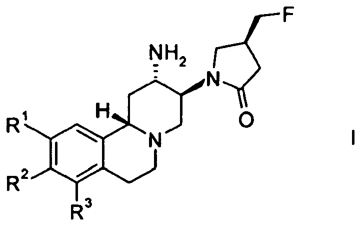 wherein R', R² and R³ are each independently hydrogen, halogen, hydroxy, lower alkyl, lower alkoxy or lower alkenyl, wherein lower alkyl, lower alkoxy and lower alkenyl may optionally be substituted by lower alkoxycarbonyl, aryl or heterocyclyl, and pharmaceutically acceptable salts thereof,
comprising
a) coupling an amine of formula III 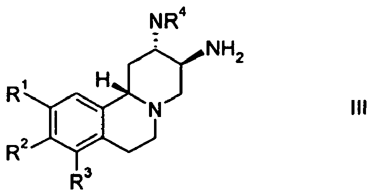 wherein R¹, R² and R³ are each independently hydrogen, halogen, hydroxy, lower alkyl, lower alkoxy or lower alkenyl, wherein lower alkyl, lower alkoxy and lower alkenyl may optionally be substituted by lower alkoxycarbonyl, aryl or heterocyclyl, and R⁴ is an amino protecting group, with the fluorolactone of the formula IV 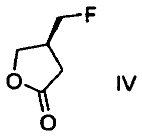 in the presence of 6-chloropyridin-2-ol as catalyst to form the butyramide of the formula V 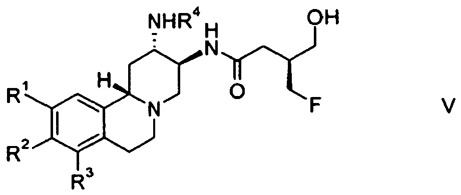 wherein R¹, R², R³ and R⁴ are as defined above;
b) forming the mesylate of formula VI 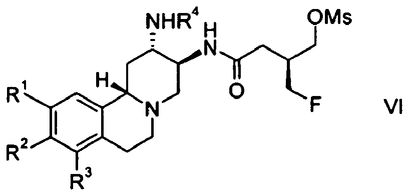 wherein R¹, R², R³ and R⁴ are as defined above and Ms stands for methanesulfonyl, by reacting the butyramide of formula V with methanesulfonyl chloride or methanesulfonyl anhydride;
c) ring closing of the mesylate of formula VI in the presence of an organic base to form the N-protected pyrido[2,1-a]isoquinoline derivative of formula II 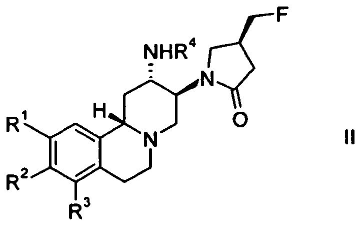 wherein R¹, R² and R³ are as defined above and R⁴ is an amino protecting group and
d) deprotecting the N-protected pyrido[2,1-a]isoquinoline derivative of formula II with hydrochloric acid in a solvent selected from the group consisting of acetone, methyl ethyl ketone, acetonitrile, tetrahydrofuran or mixtures thereof, and water in admixture with acetone, methyl ethyl ketone, acetonitrile, tetrahydrofuran or mixtures thereof, wherein at the end of step a), the amount of solvent must be reduced by distillation in order to achieve full conversion of compound of amine of formula III to butyramide of formula V,
wherein the butyramide of formula V obtained from step a) is used for the mesylation step b) without isolation from the reaction mixture,
and wherein the term "lower" is used to mean a group consisting of one to six carbon atoms.

2. Process according to claim 1, **characterized in that** acetone or tetrahydrofuran or mixtures thereof, or water in admixture with acetone or tetrahydrofuran or mixtures thereof are used.

3. Process according to claims 1 or 2, wherein R¹ and R² are methoxy and R³ is hydrogen.

4. Process according to claim 1, **characterized in that** in step a) the 6-chloropyridin-2-ol catalyst is applied in an amount of 0.05 mol equivalents to 0.20 mol equivalents relating to one mol equivalent of the amine of formula III.

5. Process according to claims 1 or 4, **characterized in that** the conversion in step a) is performed at a temperature of 80 °C to 111 °C.

6. Process according to any one of claims 1 or 4 to 5, **characterized in that** in step b) methanesulfonyl chloride is used.

7. Process according to any one of claims 1 or 4 to 6, **characterized in that** the conversion in step b) is performed at a temperature of 10 °C to 35 °C.

8. Process according to any one of claims 1 or 4 to 7, **characterized in that** the mesylate of formula VI obtained in step b) is used for the ring closing step c) without isolation from the reaction mixture.

9. Process according to any one of claims 1 or 4 to 8, **characterized in that** in step c) lithium tert.-butoxide or lithium-bis(trimethylsilyl)amide is selected as organic base.

10. Process according to claim 9, **characterized in that** lithium tert.-butoxide or lithium-bis(trimethylsilyl)amide is applied in an amount of 2.5 mol equivalents to 3.5 mol equivalents relating to one mol equivalent of the amine of formula III.

11. Process according to any one of claims 1 or 4 to 10, **characterized in that** the conversion in step c) is performed at a temperature of -20 °C to 10 °C.

12. Process according to any one of claims 1 or 4 to 11, **characterized in that** for the deprotection in step d) a solvent selected from acetone, methyl ethyl ketone, acetonitrile or tetrahydrofuran or mixtures thereof, or water in admixture with acetone, methyl ethyl ketone, acetonitrile or tetrahydrofuran or mixtures thereof are used.

13. Process according to claim 12, **characterized in that** acetone or tetrahydrofuran or mixtures thereof or water in admixture with acetone or tetrahydrofuran or mixtures thereof are used.

14. Process according to any one of claims 1 or 4 to 13, **characterized in that** the conversion in step d) is performed at a temperature of 35 °C to 66 °C.

15. Process according to any one of claims 1 to 14, wherein the compound of formula I is (2S,3S,11bS)-1-(2-amino-9,10-dimethoxy-1,3,4,6,7,11b-hexahydro-2H-pyrido[2,1-a]isoquinolin-3-yl)-4(S)-fluoromethyl-pyrrolidin-2-one dihydrochloride.

## Patentansprüche

1. Verfahren zur Herstellung eines Pyrido[2,1-a]isochinolin-Derivats der Formel 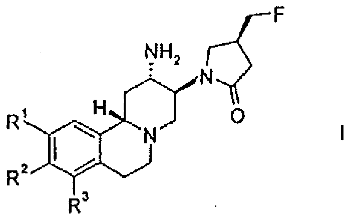 wobei R¹, R² und R³ jeweils unabhängig Wasserstoff, Halogen, Hydroxy, Niederalkyl, Niederalkoxy oder Niederalkenyl sind, wobei Niederalkyl, Niederalkoxy und Niederalkenyl gegebenenfalls mit Niederalkoxycarbonyl, Aryl oder Heterocyclyl substituiert sein können, und pharmazeutisch verträglicher Salze davon,
umfassend
a) Kuppeln eines Amins der Formel III 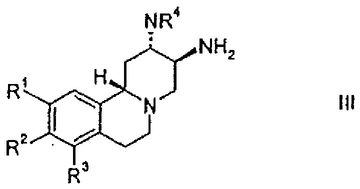 wobei R¹, R² und R³ jeweils unabhängig Wasserstoff, Halogen, Hydroxy, Niederalkyl, Niederalkoxy oder Niederalkenyl sind, wobei Niederalkyl, Niederalkoxy und Niederalkenyl gegebenenfalls mit Niederalkoxycarbonyl, Aryl oder Heterocyclyl substituiert sein können, und R⁴ eine Aminoschutzgruppe ist, mit dem Fluorlacton der Formel IV 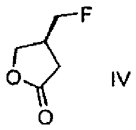 in Gegenwart von 6-Chlorpyridin-2-ol als Katalysator, um das Butyramid der Formel V zu bilden 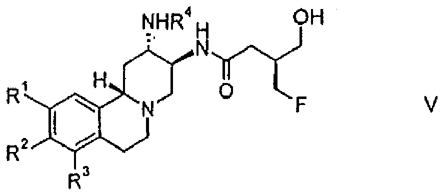 wobei R¹, R², R³ und R⁴ wie vorstehend definiert sind;
b) Bilden des Mesylats der Formel VI 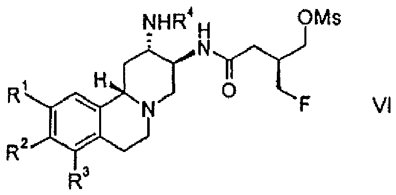 wobei R¹, R², R³ und R⁴ wie vorstehend definiert sind und Ms für Methansulfonyl steht, durch Umsetzen des Butyramids der Formel V mit Methansulfonylchlorid oder Methansulfonylanhydrid;
c) Ringschluss des Mesylats der Formel VI in Gegenwart einer organischen Base, um das N-geschützte Pyrido[2,1-a]isochinolin-Derivat der Formel II zu bilden 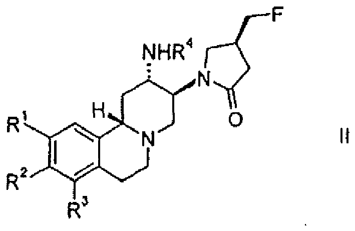 wobei R¹, R² und R³ wie vorstehend definiert sind und R⁴ eine Aminoschutzgruppe ist, und
d) Entfernen der Schutzgruppe von dem N-geschützten Pyrido[2,1-a]isochinolin-Derivat der Formel II mit Salzsäure in einem Lösungsmittel, ausgewählt aus der Gruppe bestehend aus Aceton, Methylethylketon, Acetonitril, Tetrahydrofuran oder Gemischen davon, und Wasser im Gemisch mit Aceton, Methylethylketon, Acetonitril, Tetrahydrofuran oder Gemischen davon,
wobei am Ende von Schritt a) die Lösungsmittelmenge durch Destillation verringert werden muss, um eine vollständige Umwandlung der Aminverbindung der Formel III in Butyramid der Formel V zu erzielen,
wobei das aus Schritt a) erhaltene Butyramid der Formel V ohne Isolierung aus dem Reaktionsgemisch für den Mesylierungsschritt b) verwendet wird,
und wobei der Begriff "Nieder" so verwendet wird, dass er einen aus einem bis sechs Kohlenstoffatomen bestehenden Rest bedeutet.

2. Verfahren nach Anspruch 1, **dadurch gekennzeichnet, dass** Aceton oder Tetrahydrofuran oder Gemische davon, oder Wasser im Gemisch mit Aceton oder Tetrahydrofuran oder Gemischen davon verwendet werden.

3. Verfahren nach den Ansprüchen 1 oder 2, wobei R¹ und R² Methoxy sind und R³ Wasserstoff, ist.

4. Verfahren nach Anspruch 1, **dadurch gekennzeichnet, dass** in Schritt a) der 6-Chlorpyridin-2-ol-Katalysator in einer Menge von 0,05 Moläquivalenten bis 0,20 Moläquivalenten, bezogen auf ein Moläquivalent des Amins der Formel III, verwendet wird.

5. Verfahren nach den Ansprüchen 1 oder 4, **dadurch gekennzeichnet, dass** die Umwandlung in Schritt a) bei einer Temperatur von 80°C bis 111°C durchgeführt wird.

6. Verfahren nach einem der Ansprüche 1 oder 4 bis 5, **dadurch gekennzeichnet, dass** in Schritt b) Methansulfonylchlorid verwendet wird.

7. Verfahren nach einem der Ansprüche 1 oder 4 bis 6, **dadurch gekennzeichnet, dass** die Umwandlung in Schritt b) bei einer Temperatur von 10°C. bis 35°C durchgeführt wird.

8. Verfahren nach einem der Ansprüche 1 oder 4 bis 7, **dadurch gekennzeichnet, dass** das in Schritt b) erhaltene Mesylat der Formel VI ohne Isolierung aus dem Reaktionsgemisch für den Ringschluss-Schritt c) verwendet wird.

9. Verfahren nach einem der Ansprüche 1 oder 4 bis 8, **dadurch gekennzeichnet, dass** in Schritt c) Lithium-tert.-butoxid oder Lithium-bis(trimethylsilyl)amid als organische Base ausgewählt wird.

10. Verfahren nach Anspruch 9, **dadurch gekennzeichnet, dass** Lithium-tert.-butoxid oder Lithium-bis(trimethylsilyl)amid in einer Menge von 2,5-Moläquivalenten bis 3,5 Moläquivalenten, bezogen auf ein Moläquivalent des Amins der Formel III, verwendet wird.

11. Verfahren nach einem der Ansprüche 1 oder 4 bis 10, **dadurch gekennzeichnet, dass** die Umwandlung in Schritt c) bei einer Temperatur von -20°C bis 10°C durchgeführt wird.

12. Verfahren nach einem der Ansprüche 1 oder 4 bis 11, **dadurch gekennzeichnet, dass** für das Entfernen der Schutzgruppe in Schritt d) ein Lösungsmittel, ausgewählt aus Aceton, Methylethylketon, Acetonitril oder Tetrahydrofuran oder Gemischen davon, oder Wasser im Gemisch mit Aceton, Methylethylketon, Acetonitril oder Tetrahydrofuran oder Gemischen davon verwendet werden,

13. Verfahren nach Anspruch 12, **dadurch gekennzeichnet, dass** Aceton oder Tetrahydrofuran oder Gemische davon, oder Wasser im Gemisch mit Aceton oder Tetrahydrofuran oder Gemischen davon verwendet werden.

14. Verfahren nach einem der Ansprüche 1 oder 4 bis 13, **dadurch gekennzeichnet, dass** die Umwandlung in Schritt d) bei einer Temperatur von 35°C bis 66°C durchgeführt wird.

15. Verfahren nach einem der Ansprüche 1 bis 14, wobei die Verbindung der Formel 1 (2S,3S,11bS)-1-(2-Amino-9,10-dimethoxy-1,3,4,6,7,11b-hexahydro-2H-pyrido[2,1-a]isochinolin-3-yl)-4(S)-fluormethyl-pyrrolidin-2-on-dihydrochlorid ist.

## Revendications

1. Procédé pour la préparation d'un dérivé de pyrido-[2,1-a] isoquinoléine de formule I 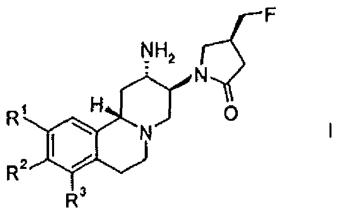 dans laquelle R¹, R² et R³ représentent chacun indépendamment un atome d'hydrogène ou d'halogène, un groupe hydroxy, alkyle inférieur, alkoxy inférieur ou alcényle inférieur, où les groupes alkyle inférieur, alkoxy inférieur et alcényle inférieur peuvent être facultativement substitués avec des substituants alkoxycarbonyle inférieur, aryle ou hétérocyclyle, et de ses sels pharmaceutiquement acceptables,
comprenant :
a) le couplage d'une amine de formule III 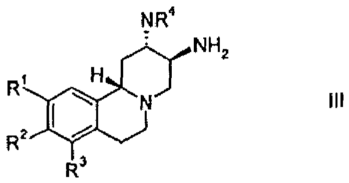 dans laquelle R¹, R² et R³ représentent chacun indépendamment un atome d'hydrogène ou d'halogène, un groupe hydroxy, alkyle inférieur, alkoxy inférieur ou alcényle inférieur, où les groupes alkyle inférieur, alkoxy inférieur et alcényle inférieur peuvent être facultativement substitués avec des substituants alkoxycarbonyle inférieur, aryle ou hétérocyclyle, et R⁴ représente un groupe protecteur de la fonction amino, avec la fluorolactone de formule IV 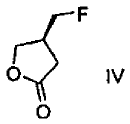 en présence de 6-chloropyridine-2-ol comme catalyseur
pour former le butyramide de formule V 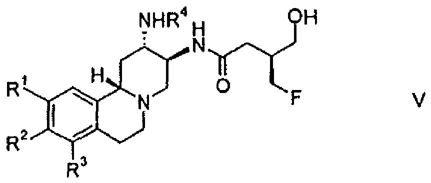 dans laquelle R¹, R², R³ et R⁴ répondent aux définitions précitées ;
b) la formation du mésylate de formule VI 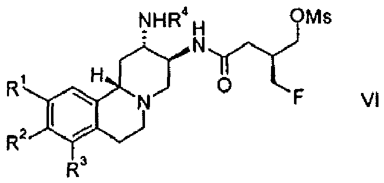 dans laquelle R¹, R², R³ et R⁴ répondent aux définitions précitées et Ms représente un groupe méthanesulfonyle, en faisant réagir le butyramide de formule V avec du chlorure de méthanesulfonyle ou de l'anhydride de méthanesulfonyle ;
c) la cyclisation du mésylate de formule VI en présence d'une base organique pour former le dérivé de pyrido [2,1-a] isoquinoléine N-protégé de formule II 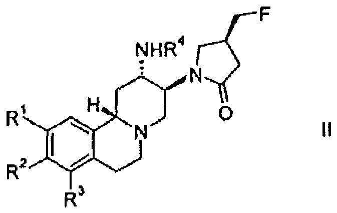 dans laquelle R¹, R², et R³ répondent aux définitions précitées et R⁴ représente un groupe protecteur de la fonction amino, et
d) à suppression de protection du dérivé de pyrido-[2,1-a] isoquinoléine N-protégé de formule II avec de l'acide chlorhydrique dans un solvant choisi dans le groupe consistant en l'acétone, la méthyléthylcétone, l'acétonitrile, le tétrahydrofurane et leurs mélanges, et de l'eau en mélange avec de l'acétone, de la méthyléthylcétone, de l'acétonitrile, du tétrahydrofurane ou leurs mélanges,
dans lequel, à la fin de l'étape a), la quantité de solvant doit être réduite par distillation afin de parvenir à la conversion totale du composé consistant en l'amine de formule III en le butyramide de formule V,
dans lequel le butyramide de formule V obtenu dans l'étape a) est utilisé pour l'étape de mésylation b) sans isolement du mélange réactionnel,
et dans lequel le terme "inférieur" est utilisé pour désigner un groupe consistant en un à six atomes de carbone.

2. Procédé suivant la revendication 1, caractérisé en que de l'acétone ou du tétrahydrofurane ou bien leurs mélanges, ou de l'eau en mélange avec de l'acétone ou du tétrahydrofurane ou bien leurs mélanges, sont utilisés.

3. Procédé suivant la revendication 1 ou 2, dans lequel R¹ et R² représentent des groupes méthoxy et R³ représente un atome d'hydrogène.

4. Procédé suivant la revendication 1, caractérisé en que, dans l'étape a), le catalyseur 6-chloropyridine-2-ol est appliqué en une quantité de 0,05 équivalent molaire à 0,20 équivalent molaire par rapport à un équivalent molaire de l'amine de formule III.

5. Procédé suivant la revendication 1 ou 4, caractérisé en que la conversion dans l'étape a) est effectuée à une température de 80°C à 111°C.

6. Procédé suivant l'une quelconque des revendications 1 ou 4 et 5, caractérisé en que, dans l'étape b), du chlorure de méthanesulfonyle est utilisé.

7. Procédé suivant l'une quelconque des revendications 1 ou 4 à 6, caractérisé en que la conversion dans l'étape b) est effectuée à une température de 10°C. à 35°C.

8. Procédé suivant l'une quelconque des revendications 1 ou 4 à 7, caractérisé en que le mésylate de formule VI obtenu dans l'étape b) est utilisé pour l'étape de cyclisation c) sans isolement du mélange réactionnel.

9. Procédé suivant l'une quelconque des revendications 1 ou 4 à 8, caractérisé en que, dans l'étape c), du tertiobutylate de lithium ou du bis(triméthylsilyl)amidure de lithium est choisi comme base organique.

10. Procédé suivant la revendication 9, caractérisé en que du tertiobutylate de lithium ou du bis(triméthylsilyl)-amidure de lithium est appliqué en une quantité de 2,5 équivalents molaires à 3,5 équivalents molaires par rapport à un équivalent molaire de l'amine de formule III.

11. Procédé suivant l'une quelconque des revendications 1 ou 4 à 10, caractérisé en que la conversion dans l'étape c) est effectuée à une température de -20°C à 10°C.

12. Procédé suivant l'une quelconque des revendications 1 ou 4 à 11, caractérisé en que, pour la suppression de protection dans l'étape d), un solvant choisi entre l'acétone, la méthyléthylcétone, l'acétonitrile ou le tétrahydrofurane ou bien leurs mélanges, ou de l'eau en mélange avec de l'acétone, de la méthyléthylcétone, de l'acétonitrile ou du tétrahydrofurane ou bien leurs mélanges, est utilisé.

13. Procédé suivant la revendication 12, caractérisé en que de l'acétone ou du tétrahydrofurane ou bien leurs mélanges ou de l'eau en mélange avec de l'acétone ou du tétrahydrofurane ou bien leurs mélanges sont utilisés.

14. Procédé suivant l'une quelconque des revendications 1 ou 4 à 13, caractérisé en que la conversion dans l'étape d) est effectuée à une température de 35°C à 66°C.

15. Procédé suivant l'une quelconque des revendications 1 à 14, dans lequel le composé de formule I est le dichlorhydrate de (2S,3S,11bS)-1-(2-amino-9,10-diméthoxy-1,3,4,6,7,11b-hexahydro-2H-pyrido[2,1-a]isoquinoléine-3-yl)-4(S)-fluorométhyl-pyrrolidine-2-one.
